# FASCICULE DE BREVET EUROPEEN

(11) **EP 0 752 865 B1**
(45) Date de publication et mention de la délivrance du brevet: **20.11.2002**
(21) Numéro de dépôt: 95915225.7
(22) Date de dépôt: 29.03.1995
(51) Int. Cl.: A61K 31/715, A61K 31/727, A61P 25/00, A61P 25/28

(54) **UTILISATION DE DEXTRANE SUBSTITUE POUR LE TRAITEMENT DES LESIONS DU SYSTEME NERVEUX ET FRACTIONS ENRICHIES EN HERAPANE SULFATE**
VERWENDUNG VON SUBSTITUIERTEN DEXTRANEN ZUR BEHANDLUNG VON SCHÄDIGUNGEN DES NERVENSYSTEMS, UND FRAKTIONEN MIT ERHÖHTEM HEPARANSULFAT-GEHALT
USE OF SUBSTITUTED DEXTRANE FOR TREATING NERVOUS SYSTEM DAMAGE, AND HEPARANE SULPHATE-ENRICHED FRACTIONS

(30) Priorité: 30.03.1994 FR 9403806
(43) Date de publication de la demande: 15.01.1997
(73) Titulaire: Valbiofrance, 75006 Paris (FR)
(72) Inventeur: BARRITAULT, Denis, F-75001 Paris (FR); CARUELLE, Jean-Pierre, F-94100 Saint-Maur (FR); AAMIRI, Ahmed, Agadir (MA); GAUTRON, Jean, F-94100 Vitry-sur-Seine (FR)
(74) Mandataire: Michelet, Alain
(86) Numéro de dépôt international: FR9500401
(87) Numéro de publication internationale: WO95026739

(56) Documents cités:
- EP-A- 0 269 937
- FR-A- 2 461 724
- FR-A- 2 644 066
- MINERVA DIETOL. GASTROENTEROL., vol.31, no.2, pages 311 - 315 A. SAGGIORO ET AL. 'Trattamento della crisi emorroidaria con mesoglicano solfato.'

## Description

La présente invention a pour objet l'utilisation de polymères ou de biopolymères pour la préparation d'un médicament pour le traitement de lésions de toutes origines affectant le système nerveux central ou périphérique en médecine humaine ou vétérinaire. Elle est en outre relative à des compositions pharmaceutiques pour ce traitement. Elle a enfin pour objet des fractions enrichies en héparane sulfate.

La synthèse des polymères CMDBS (dextranes substitués par des résidus carboxyméthyle, benzylamine et sulfonate) a été décrite dans le brevet FR 2461724 ainsi que dans le brevet US 4 740 594. Certains de ces polymères miment l'héparine et peuvent être utilisés en tant que produits de remplacement de l'héparine du plasma, grâce à leurs propriétés anticoagulante et anticomplément.

Parmi l'ensemble des polymères CMDBS, certains de ces polymères miment une autre propriété de l'héparine qui consiste en une stabilisation, une protection et une potentialisation de l'activité biologique in vitro des facteurs de croissance de la famille FGF (Tardieu et coll , Journal of Cellular Physiology,1992,150 Pages 194 à 203).

Le brevet FR 2 644.066 décrit l'utilisation de certains CMDBS associés aux FGF pour la cicatrisation de la peau et de la cornée. Des expériences ont été réalisées en provoquant une blessure cutanée à l'aide d'un emporte pièce de 6 mm de diamètre chez le rat. Dans cet exemple, le CMDBS associé au FGF 2 permet d'obtenir un effet net sur la vitesse et la qualité de la réparation de la peau.

Un autre biopolymère, le dextrane sulfate a également été proposé en association avec des FGF, comme stabilisateur et protecteur, dans le brevet Japonais N°13890. Le dextrane sulfate est par ailleurs largement utilisé dans des pommades ou crèmes cicatrisantes de la peau ainsi que dans des compositions de collyre, mais n'a aucun effet rapporté à la connaissance du demandeur sur la cicatrisation et ou la régénération de lésions du système nerveux.

Un nombre important de facteurs ayant des activités facilitant la survie des cellules nerveuses, la réparation de lésions nerveuses centrales ou périphériques, ainsi que les réinnervations musculaires ont déjà été décrits: les facteurs polypeptidiques comme le Facteur de Croissance des Nerfs (NGF), les facteurs des familles FGF, les facteurs dérivés du cerveau comme les BDNF, le Ciliary Neurotrophic factor (CNTF), le Neurotrophin 3 (NT3) etc.

Ces facteurs ont été utilisés dans des expériences de réinnervation musculaires, de réparation de coupures de nerfs périphériques, moteurs, dans des modèles de lésions de cellules nerveuses centrales cholinergiques, ainsi que dans de nombreux autres modèles. A titre de références, les revues mentionnées ci-après décrivent une partie de ces travaux: P.M. Richardson, Current Opinion in Neurobiology, 1991, 1: 401-406 ; T. Ebendal, Journal of Neurosciences Research, 1992, 32: 461-470 ; P.G. Cordeiro, R. Brooke et al., Plastic and Reconstructive Surgery, 1989, 86(3): 1013-1019 ; Q. Yan, J. Elliott et al., Nature (Letters to Nature), 1992, 360: 753-755 ; N.A. Seniuk, Journal of Reconstructive Microsurgery, 8 (5): 399-404 ; F. Hefti, P.P. Michel et al., Advances in Neurology, 1990, 53: 123-127 ; A.C. Cuello, L. Garofalo et al., Progress in Brain Research, 1990, 84: 301-311 ; A. Takeda, H. Onodera et al., Brain Research, 1992, 569: 177-180.

Il ressort donc de l'analyse de l'état de la technique que des facteurs de croissance et des polymères en association avec des facteurs de croissance avaient déjà été utilisés dans des applications thérapeutiques.

Néanmoins, aucun des documents cités ne montre que les polymères présentent des effets en eux-mêmes, c'est-à-dire sans qu'ils soient associés à des facteurs de croissance.

En outre, l'activité d'associations polymères-facteurs n'a été décrite que sur certaines lésions d'un type bien précis de tissu, tel que le tissu cutané.

Du fait de l'imprévisibilité des effets thérapeutiques d'une molécule donnée, il n'était pas évident que ces polymères puissent avoir un effet sur d'autres tissus.

En effet, il est bien connu que les différents tissus du corps humain ou animal présentent des spécificités tant structurelles que fonctionnelles qui rendent impossible toute prédiction quant à l'effet d'une molécule, connue pour son effet sur le tissu cutané sur un autre tissu.

De même, il est bien connu qu'il est impossible de prédire l'activité in vivo d'une molécule sur un tissu particulier à partir de résultats obtenus in vitro sur un modèle expérimental spécifique.

De manière surprenante, il a été trouvé, selon l'invention, que certains CMDBS définis comme appartenant à la classe des HBGFPP ont un effet très marqué sur la vitesse de cicatrisation et de régénération des lésions des tissus nerveux centraux ou périphériques ainsi que sur la qualité de cette cicatrisation et/ou régénération telle que l'on peut la mesurer en l'étudiant par des méthodes histologiques et physiologiques. La réinnervation musculaire avec réformation d'une jonction fonctionnelle du nerf lésé avec son muscle a été observée.

La présente invention a pour objet l'utilisation d'au moins un polymère ou d'un biopolymère, appelés HBGFPP, protégeant spécifiquement les facteurs de croissance des familles des FGF et TGF bêta de la dégradation trypsique et n'inhibant pas de manière significative la coagulation, pour la fabrication d'un médicament pour le traitement des tissus musculaires.

Un tel polymère présente particulièrement une activité anti-coagulante inférieure à 50 unités internationales par mg de polymère mesurée selon Maillet et al. (Mol. Immunol, 1988, 25, 915-923). Préférentiellement, il n'active substantiellement pas le système du complément, c'est-à-dire qu'il possède une activité anti-complément supérieure à 0,5 µg pour le CH5O (selon Mauzac et al., Biomaterials, 6, 61-63, 1985).

Avantageusement, il potentialise in vitro les FGF.

Selon la présente invention on entend par polymères toutes substances naturelles, naturelles modifiées chimiquement ou totalement synthétiques répondant à la définition donnée ci-dessus.

Ainsi il peut s'agir de :
- polymères obtenus à partir de dextranes mais modifiés par d'autres types de substitutions avec d'autres types de radicaux,
- polymères naturels autres que ceux dérivant de dextranes mais comportant des résidus osidiques (cellulose, chitine, fucanes, etc...),
- polymères obtenus par polymérisation de monomères de natures non osidiques (poly acide malique, poly acide oxalique, poly acide lactique, polystyrène, polyéthylène glycol) modifiés ou non.

Avantageusement, ledit polymère ou biopolymère est un polysaccharide qui peut être composé principalement de résidus glucose.

Un tel polysaccharide présentera préférentiellement un poids moléculaire supérieur à 10 kD et avantageusement proche de 40 kD.

Il peut aussi comprendre des résidus glucosamine et/ou d'acide uronique, particulièrement sous la forme de dimère glucosamine-acide uronique.

Des polysaccharides particulièrement préférés sont des dextranes substitués, des glycosaminoglycanes éventuellement associés à un lipide, un peptide ou un protide ou des sulfates de ces polymères.

La présente invention est en outre relative à une composition pharmaceutique contenant de ces polymères.

Les polymères et/ou biopolymères peuvent être séléctionnés à partir de substances naturelles qui peuvent ensuite être éventuellement modifiées par additions de groupements chimiques appropriés ou encore obtenus entièrement par synthèse . Ces polymères naturels, semi synthétiques ou entièrement synthétiques sont ensuite sélectionnés sur la base de leurs capacités à interagir spécifiquement avec plusieurs facteurs de croissance notamment ceux de la famille des FGF et des TGF bêta. Ils sont également sélectionnés sur leurs capacité à protéger ce ou ces facteurs contre des dégradations protéolytiques. Ces polymères seront désignés sous le sigle générique de HBGFPP (heparin binding growth factor protectors and promotors).

Deux prototypes de ces polymères ou bio polymères sont donnés comme exemples ainsi que les procédés et critères de sélection de ces polymères.

Le premier exemple de HBGFPP appartient à la famille des CMDBS qui sont des produits connus, à savoir des dextranes biospécifiques fonctionnalisés, substitués par des résidus carboxyméthyle, benzylamide et benzylamine sulfonate. Ces polymères illustrent l'obtention de HBGFPP à partir de produits naturels (dextrans) subséquemment chimiquement substitués.

Le deuxième exemple décrit la sélection de produits complètement naturels comme les protéoglycosaminoglycanes sulfates purifiés à partir d'extraits tissulaires.

Ces deux exemples illustrent les capacités de ces HBGFPP à interagir, à stabiliser, à protéger et à potentialiser les facteurs de croissances des familles FGF et TGF bêta et leur utilisation dans une composition pharmaceutique permettant une cicatrisation et une régénération des liaisons nerveuses ainsi qu'une protection et cicatrisation des cellules nerveuses.

On entend , dans la présente demande, par traitement, toute opération curative ou préventive effectuée pour la prophylaxie, la cicatrisation, la protection ou la régénération de lésions touchant le système nerveux.

Grâce à l'action des HBGFPP et notamment des CMDBS comme l'illustrent les exemples ci-dessous , la réinnervation du muscle de type EDL ou Soleus est accélérée. Cette réinnervation se traduit par la régénération de la fibre nerveuse et la reformation rapide d'une jonction synaptique fonctionnelle.

On observe lors des lésions proches du muscle non seulement une augmentation de la croissance axonale mais également un contrôle de cette croissance. Ainsi au niveau du bourgeonnement ("sprouting"), cette croissance se produit d'une manière organisée et orientée se traduisant par une accélération de la réparation fonctionnelle.

Les propriétés des HBGFPP font de cette famille de molécules une classe tout à fait nouvelle et unique de médicaments utilisables pour favoriser et améliorer les lésions du système nerveux central ou périphérique, touchant directement les cellules neuronales et leurs prolongements axonaux et dendritiques, les neurones cholinergiques ou dopaminergiques ou encore touchant les cellules associées aux neurones comme les cellules gliales astrocytaires oligodendrocytes et cellules de Schwann. Les causes de ces lésions peuvent être de toutes origines: traumatique, iatrogène ou chimique, liées à l'utilisation de rayonnements ou induites par des interventions chirurgicales, d'origines infectieuses bactériennes, parasitaires, virales ou encore d'origine auto-immune ou encore, des lésions et détériorations induites par des saignements comme des ruptures de vaisseaux. Ces nouveaux médicaments s'utilisent également pour le traitement de maladies neurodégénératives comme les maladies de Parkinson ou d'Alzheimer ou d'origines génétiques. Enfin, ces médicaments peuvent être associés avec bénéfice aux cellules utilisées dans les thérapies de transplantations dans les zones atteintes du cerveau à l'aide de cellules normales ou modifiées génétiquement.

Le médicament et la composition pharmaceutique selon l'invention peuvent contenir une quantité efficace de HBGFPP par exemple du CMDBS associé à un ou plusieurs véhicules compatibles et pharmaceutiquement acceptables. Elle peut être également associée à des agents pharmaceutiques comme des anti-inflammatoires et/ou des antibactériens.

Le véhicule peut être du sérum physiologique ou des tampons tels que le PBS contenant NaCl 0,15 M ou toute autre sorte de solution compatible et non irritante pour les tissus nerveux lésés. Des formulations permettant d'obtenir des solutions pâteuses ou en gel selon les techniques courantes connues de l'homme de l'art peuvent être proposées selon le type et l'accessibilité de la lésion.

Avantageusement, une telle composition est conçue pour être directement injectable directement sur le site de la lésion à une dose de 2,5 à 2500 mg/ml de HBGFPP comme le CMDBS dans les exemples donnés ou comme les biopolymères naturels HBGFPP tels le mésoglycan mais la voie intraveineuse ou intramusculaire peut être préférée dans le cas de désinnervation de muscle désinnervé. L'injection dans la moelle épinière peut être également préférable dans le cas de lésions de celle-ci ou de lésions au niveau des embranchements de prolongements de fibres nerveuses qu'elles soient motrices et/ ou sensitives. Le volume d'injection est estimé en fonction de l'importance du volume de la lésion. Des doses correspondant à 100 µl sont souvent suffisantes.

Outre ses qualités de protecteur des facteurs de croissance "Heparin Binding", les HBGFPP sélectionnés selon les tests décrits ci-dessous présentent une très faible activité anticoagulante, par rapport à celle de l'héparine, trop faible pour perturber la coagulation dans le cas d'un traumatisme. Dans le cas d'une injection par voie intraveineuse, la dose injectée doit être rapportée au volume sanguin de l'homme ou de l'animal ainsi traité pour que la dose de HBGFPP dans le sang soit également comprise entre 2,5 à 2500 mg /ml.

A titre d'exemples décrits dans les pages suivantes et concernant la réinnervation du muscle squelettique du rat adulte de type ( Extensor Digitorum longus (EDL) ou des muscles lents posturaux (Soleus), une injection unique de 100 µl d'une solution de CMDBS à 50 µg/ml sur le site de la blessure induit une réinnervation complète en 17 jours alors que dans le cas du muscle EDL, cette réinnervation ne s'observe qu'en 60 jours sans traitement. Dans le cas des muscles Soleus, l'effet du CMDBS est encore plus marqué puisque après injection de CMDBS, la réinnervation est totale et fonctionnelle en 17 jours alors que la réinnervation du muscle controlatéral est défectueuse même après 60 jours. Ces effets sont spécifiques des HBGFPP et notamment de certains CMDBS répondant aux critères de sélection vis-à-vis de la protection des FGFs et des facteurs de croissance de la famille TGF bêta contre les dégradations protéolytiques induites par l'action de la trypsine. Cette spécificité peut s'illustrer en comparant les effets de réparation des lésions des tissus nerveux par les HBGFPP et des produits voisins comme l'héparine, le dextrane, le dextrane sulfate ou la Sucrase (sucrose octyl sulfate). Bien que ces molécules interagissent avec les FGFs et tout au moins en ce qui concerne l' héparine avec le TGF bêta , ni la sucrase, ni l'héparine, ni le dextrane sulfate ne protègent le TGF bêta contre la protéolyse induite par l'action de la trypsine ainsi que le montre l'application des tests de criblages et de sélection des HBGFPP décrits dans les exemples ci-dessous. Ces produits sont sans effet sur les lésions des tissus nerveux. Ainsi, en procédant à un criblage in vitro sur la base d'une protection double des FGFs et des TGFbêta contre l'action de la protéolyse induite par la trypsine il est possible de séléctionner des HBGFPP comme certains CMDBS donnés dans les exemples. Ces mêmes critères de sélection appliqués à des biopolymères naturels comme le mésoglycan ou le sulodexide ont permis de montrer que le mésoglycan qui présente une double activité de protection et de stabilisation pour à la fois les FGF et les TGFbêta présente une activité favorisante pour la réparation et la régénération nerveuses et à ce titre fait partie de la famille des HBGFPP alors que le sulodexide qui protège les FGFs contre la protéolyse induite par l'action de la trypsine n'a pas d'action protectrice significative contre l'action de la trypsine sur les TGF bêta.

La présente invention est en outre relative à des fractions du mésoglycan ou du sulodexide enrichies en héparane sulfate, présentant avantageusement 80%, et préférentiellement 95%, d'héparane sulfate.

De telles fractions peuvent être obtenues par un procédé d'enrichissement en héparane sulfate d'une composition de glycosaminoglycanes, comprenant les étapes suivantes:
- chromatographie échangeuse d'ions de la composition,
- élution sur un gel de DEAE,
- traitement par la chondroïtinase ABC,
- chromatographie sur tamis moléculaire, et
- élution de l'héparane sulfate.

L'invention sera illustrée, sans être aucunement limitée par les exemples qui suivent, dans lesquels:
La figure 1 représente la formule du CMDBS.
La figure 2 illustre la potentialisation de l'activité biologique des FGF1 (2a) et FGF2 (2b) par l'héparine, le mésoglycan et le sulodexide. La mesure de l'activité biologique est effectuée sur des cellules CCL39 par la mesure de l'augmentation de l'incorporation de thymidine tritiée en fonction de la dose de FGF1 et de FGF2 ajoutée seule ou en présence de 20 µg d'héparine , de 10 µg de mésoglycan ou de 10 µg de sulodexide.
Les figures 3 et 4 illustrent l'effet protecteur de l'héparine, du mésoglycan et du sulodexide contre une dégradation thermique du FGF1(3) et FGF2(4). Les échantillons de FGF sont incubés seuls ou en présence de 20 µg d'héparine , de 10 µg de mésoglycan ou de 10 µg de sulodexide à 20°C (a) et 37°C (b) pendant 1, 7, 15, 30 jours. La mesure de l'activité biologique présentée en abscisse correspond aux valeurs des unités de stimulation (ED5O) de l'incorporation de thymidine tritiée dans des cellules CCL39.
La figure 5a illustre l'effet protecteur de l'héparine, du mésoglycan et du sulodexide contre une dégradation protéolytique du ¹²⁵I-FGF1. La digestion protéolytique a été effectuée à 37°C et les échantillons ont été séparés par électrophorèse sur gel de polyacrylamide à 18 %. Les gels sont séchés et autoradiographiés. La première piste contient le ¹²⁵I-FGF1 seul, dans la deuxième (piste 2) le ¹²⁵I-FGF1 est incubé en présence de trypsine et d'héparine (piste 3), de mésoglycan (piste 4) ou de sulodexide (piste 5).
La figure 5b illustre l'effet protecteur de l'héparine, du mésoglycan et du sulodexide contre une dégradation protéolytique du ¹²⁵I-FGF2. La disposition des pistes est identique à celle présentée pour le ¹²⁵I-FGF1 en 5a.
Les figures 6A et 6B sont des profils d'élution sur colonne de DEAE-Trisacryl respectivement des fractions HSM (Fig. 6A) et HSS (Fig. 6B), en présence de fractions chondroïtines sulfates (CSA) pour le calibrage de la colonne.
Les figures 7A à 7F représentent la mise en évidence des gouttières synaptiques des plaques motrices (P) par l'Acétyl Choline Estérase (AChE). Les axones (A) sont révélés en brun-noir par l'imprégnation argentique sur des coupes longitudinales de 80 mm d'épaisseur.

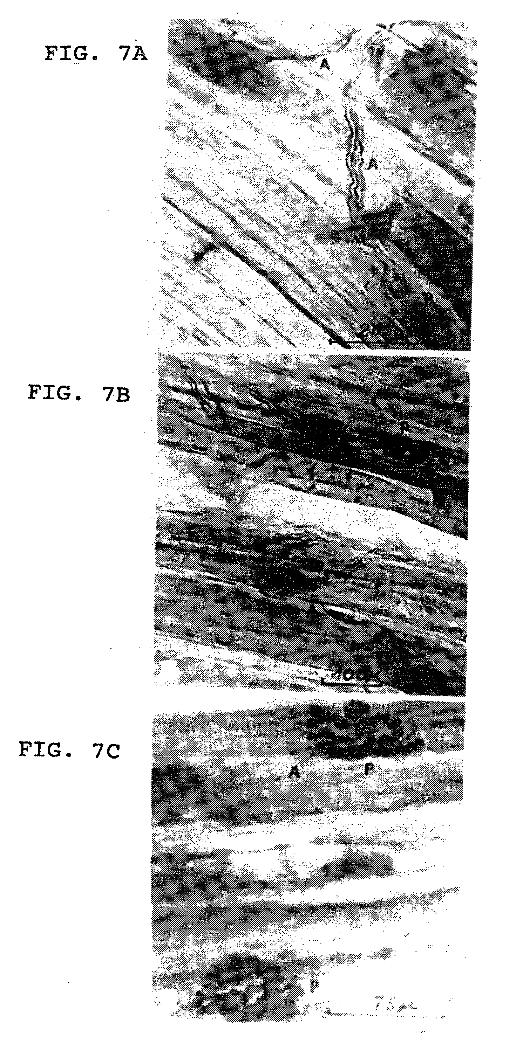

La figure 7A représente une coupe de muscle EDL normal adulte. Les plaques motrices des fibres rapides ont une gouttière synaptique très développée et différenciée (en haut à gauche) (x 150).
La figure 7B représente une coupe de muscle EDL après 60 jours de régénération sans traitement. Les plaques sont bien reformées, elles ont un diamètre analogue à celui du témoin homologue mais la surface et la différenciation synaptique sont plus faibles (x 150).
La figure 7C représente une coupe de muscle EDL après 17 jours de régénération, traité par le CMDBS. Le diamètre de la plaque est comparable aux précédents mais la surface et la différenciation synaptique sont nettement plus élevées. (x 450).

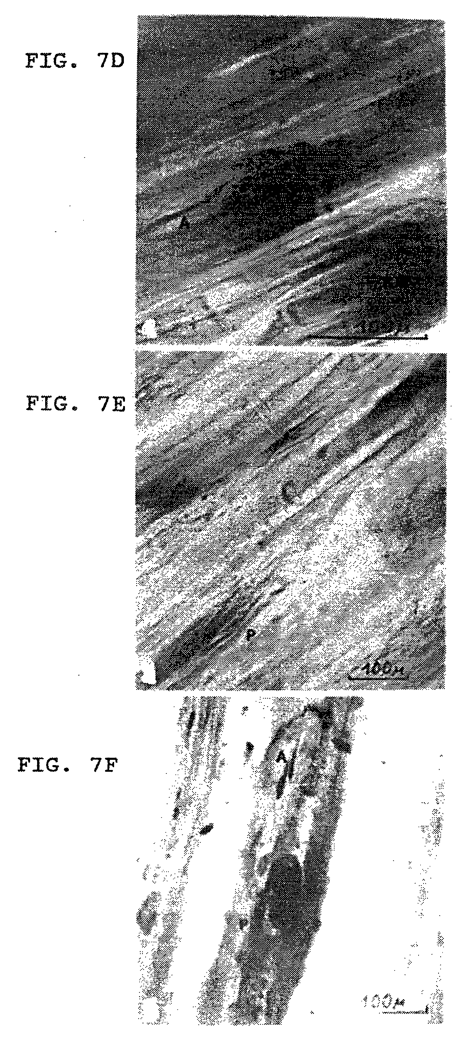

La figure 7D représente une coupe de muscle Soleus normal adulte. Le diamètre de la plaque est identique à celui de l'EDL mais la différenciation de la gouttière est moins élevée (x 300).
La figure 7E représente une coupe de muscle Soleus après 60 jours de régénération, non traité. L'AChE marque l'emplacement des plaques qui ont un aspect diffus, sans gouttière visible et déplissées. Les axones moteurs ne sont pas visibles. Ces observations sont en faveur d'une non réinnervation. (x 150).
La figure 7F représente une coupe de muscle Soleus après 17 jours de régénération, traité par le CMDBS. La gouttière synaptique est bien différenciée et fortement marquée par l'AChE. Les axones sont visibles au niveau de la plaque motrice. (x 300).

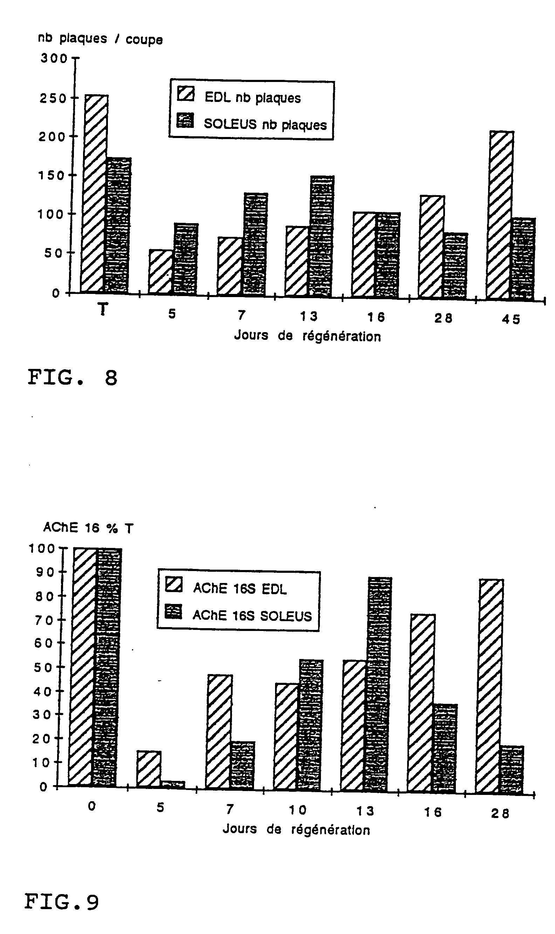

La figure 8 illustre l'évolution du nombre de plaques motrices (en ordonnée) par coupe longitudinale de muscle EDL et Soleus au cours de la régénération post-traumatique (en abscisse).
La figure 9 illustre l'évolution de la proportion (en ordonnée) de l'isoforme synaptique A12 (16S) de l'AChE dans les muscles EDL et Soleus non traités au cours de la régénération (en abscisse).

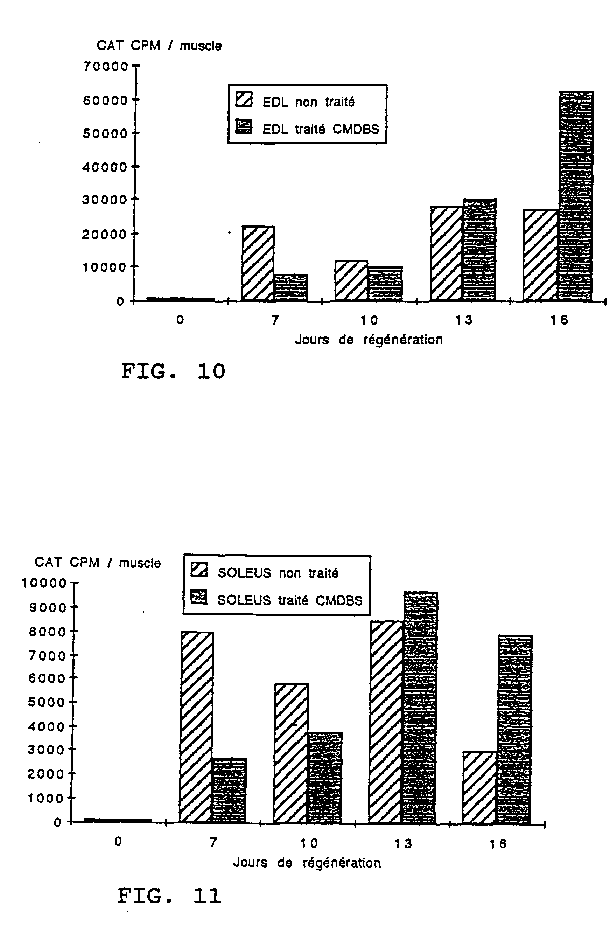

La figure 10 illustre l'activité de la choline acétyl transférase (CAT) (en ordonnée) du muscle EDL de rat adulte au cours de la régénération (en abscisse).
La figure 11 illustre l'activité de la choline acétyl transférase (en ordonnée) du muscle Soleus de rat adulte au cours de la régénération (en abscisse).

### EXEMPLE 1

### Préparation et sélection des CMDBS

### a) Préparation des CMDBS

Les CMDBS sont des dextranes substitués par des groupements carboxymethyl, benzylamide et benzylamide sulfonate. La méthode de synthèse des CMDBS peut être celle décrite par M.MAUZAC et J.JOSEFONVICZ dans Biomaterials 1984,5,301-304. Selon ce procédé, le carboxylméthyle dextrane (CMD) est préparé à partir de dextrane par substitution de quelques unités glycosylées avec des groupes carboxyliques sur le carbone en position 5 ou 6. Dans une deuxième étape, la benzylamide est couplée aux groupes carboxyliques pour former le carboxyméthyl-benzylamide dextrane (ou CMBD). Enfin quelques noyaux aromatiques du benzylamide sont sulfonés pour aboutir au carboxyméthyle dextrane benzylamide sulfonate ou CMDBS.

Les sels de sodium de ces dérivés sont ultrafiltrés, lyophilisés et dissous dans le tampon approprié avant utilisation.

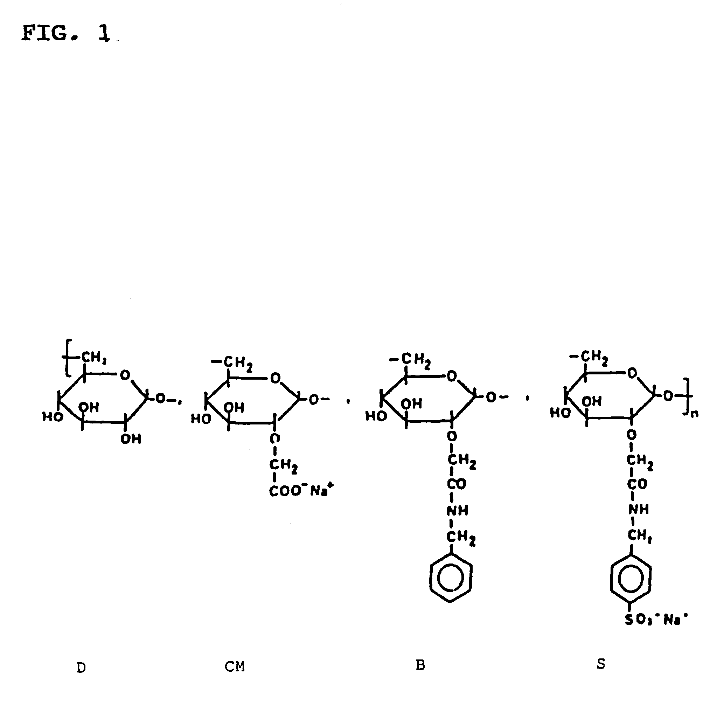

La formule générale des CMDBS est illustrée sur la figure 1.

Les CMDBS possèdent une distribution statistique des différents substituants. Les pourcentages pour chaque type de CMDBS sont déterminés par les méthodes classiques.

### b) Sélection des CMDBS

### i:Tests de protection et de stabilisation des FGFs

Lors de la synthèse des CMDBS il est possible de contrôler le taux de substitution de chacun des groupements par modification des conditions de la réaction de substitution. Le contrôle des paramètres comme la température, le temps de réaction, les concentrations relatives des constituants et le nombre de réaction de substitution etc... permettent d'obtenir un très grand nombre de polymères substitués. La substitution des hydroxyles par le carboxyméthyle sur les carbones en positions 5 et 6 permet d'obtenir des taux de carboxyméthylation allant de 0 à 200% (100% pour chacun des carbones en position 5 et 6). Le groupement carboxyméthyle peut être à son tour partiellement ou totalement utilisé pour la fixation de la benzylamide. Les groupes benzylamides peuvent être partiellement ou totalement utilisés pour la sulfonation. Les dextranes substitués fonctionnalisés utilisés selon l'invention sont parmi ceux spécialement décrits dans le brevet français n°2.461.724. Outre la capacité à stabiliser et protéger les facteurs de croissance de la famille FGF comme décrit dans la publication de Tardieu et coll J.Cell.Physio.1992 150 p 194 à 203 ; et dans le brevet Français N°2.461.724; le CMDBS sélectionné doit pouvoir interagir avec au moins un membre de la famille des facteurs de croissance de la famille TGF bêta selon une méthode d'évaluation décrite ci-dessous et protéger les TGFbêta contre une protéolyse.

### ii. Evaluation des capacités d'interactions entre CMDBS et facteurs de croissance de la famille TGF bêta.

Afin de mesurer la capacité de certains CMDBS à interagir avec les membres de la famille TGF bêta et de par cette interaction protéger les TGF bêta, un test de criblage a été établi. Ce test consiste à mesurer la capacité du CMDBS sélectionné à permettre au TGF bêta de garder son activité biologique malgré un traitement protéasique.

Dans l'exemple ci-dessous le CMDBS utilisé est le lot 26.2 défini par un taux de substitution de 110% de motifs carboxyméthyles, 3,6% de motifs benzylamides et 36,5% de motifs sulfonates et possède une activité anti-coagulante de 4 UI/mg (Unités Internationales). L'activité anti-complément de ce lot est de 1,1 µg de CH5O; elle est mesurée selon Mauzac et al.(précédemment cités).

L'héparine utilisée comme témoin provient des établissements Sanofi.(Institut Choay) et présente une activité anticoagulante de 175 UI/mg

Le TFG bêta 1 est préparé à partir de plaquettes sanguines humaines selon le protocole décrit dans de nombreuses publications et couramment utilisés par l'homme de l'art ( par exemple dans la publication Growth Factors and their Receptors 1992 , vol 1 pages 419-472 par A. Roberts et M.Sporn édité par A. Roberts et M.Sporn et publiée par Springer Verlag Berlin. Le test d'activité biologique du TGF bêta utilisé dans cet exemple est celui de l'inhibition de croissance des cellules CCL64 (provenant de l'American Tissue Culture Collection). Cette inhibition est mesurée par la capacité du TGF bêta à inhiber l'incorporation de Thymidine tritiée d'une manière dose dépendante dans ces cellules CCL64 stimulées par le facteur de croissance FGF ou par du sérum de veau foetal selon le protocole décrit par Van Zolen dans Progress in Growth Factor Research, 1990 ,2 p131 à 152.

Le TGF bêta est utilisé à deux doses, l'une correspondant à la capacité d'inhibition de 50% de l'incorporation de Thymidine tritiée (définie comme l'unité d'activité inhibitrice) l'autre, correspondant à la capacité d'inhibition de 100%. Dans cet exemple les valeurs obtenues sont de 250 pg de TGF bêta pour obtenir l'unité d'activité d'inhibition sur les cellules CCL64 cultivées dans 1 ml de milieu de culture. Le 100% d'inhibition est obtenu avec 1ng de TGF bêta.dans 1 ml de milieu de culture.

Un échantillon de 50ng de TGF bêta dans du tampon phosphate salin contenant 0.1% de sérum albumine bovine (provenant de la Société SIGMA à Saint Louis USA) est incubé seul, ou associé soit à 5000 µg de CMDBS, soit à 5000µg d'héparine, avec ou sans 500 µg de trypsine. Le volume final de la solution incubée est ajusté à 1 ml et l'incubation est effectuée à 37°C durant un temps variable (10 minutes dans l'exemple décrit ( tableau 1).

Des échantillons d'un volume de 20 µl de chacune des réactions d'incubation sont prélevés et ajoutés aux cellules CCL64 cultivées dans des plateaux de 24 puits contenant chacun un millilitre de milieu de culture selon le protocole décrit par E.Zohlen mentionné ci dessus. Dans ces conditions la concentration finale de TGF bêta par puits est de Ing/ml. Le tableau 1 résume les résultats obtenus dans diverses conditions et montre l'effet protecteur du CMDBS. Ainsi après 10 mn d'incubation à 37°C, 75% de l'activité biologique du TGF bêta est encore présente, alors que l'héparine qui pourtant peut se fixer au TGF b (Mac Caffrey et al., J. of Cell Physiology, 1992 , vol. 52, 430-440) ne protège pas le TGF bêta contre cette dégradation protéolytique (il reste moins de 20% d'activité biologique). Il est à rappeler que dans le cas des FGFs l'héparine assure une protection contre la protéolyse induite par la trypsine.(Tardieu et al., Journal of Cellular Physiology, 1992, 150: 194-203).

Il a été vérifié que le CMDBS n'avait pas de pouvoir inhibiteur sur l'activité de la trypsine (tableau 2). Ainsi, 10 µg de trypsine ont été incubés soit avec un substrat (S.87 fourni par la société Serbio, Paris et utilisé selon les recommandations de ce fournisseur) ou soit avec ce substrat et un inhibiteur de la trypsine tel celui provenant du soja (comme le Soyabean trypsin inhibitor ou STI de chez Sigma) ces incubations étant faites en l'absence ou en présence de quantités variables de CMDBS (lot AM26). L'activité enzymatique de la trypsine a été mesurée par absorption spectrophotométrique du produit de transformation du S 87 en fonction du temps d'incubation.

### EXEMPLE 2:

### Sélection d'autres HBGFPP

Deux préparations commerciales de protéoglycosaminoglycanne et glycosaminoglycannes ont été sélectionnées selon leurs capacités à interagir avec les facteurs de croissance de la famille du FGF ainsi qu'avec ceux de la famille du TGF bêta.

Des préparations d'héparane sulfate obtenues par fractionnement du mésoglycan et du sulodexide ont d'autre part été testées.

Le Mésoglycan et le Sulodexide ont été fournis par la Société Sigma Chemical Co , Saint Louis MO USA précédemment cités.

Les cellules utilisées dans cet exemple sont les cellules CCL39 qui proviennent de l'American Tissue Culture Collection. Les conditions de culture et de tests de mesure d'activité biologique des FGFs sont les mêmes que celles décrites dans la publication Tardieu et coll J.Cell.Physiol. 1992. Leurs propriétés sont résumées dans le tableau 3. Les facteurs de croissance FGF utilisés sont les formes recombinantes FGF1 et FGF 2.

### a) Effet du Mésoglycan et Sulodexide sur l'activité biologique des FGFs in vitro.

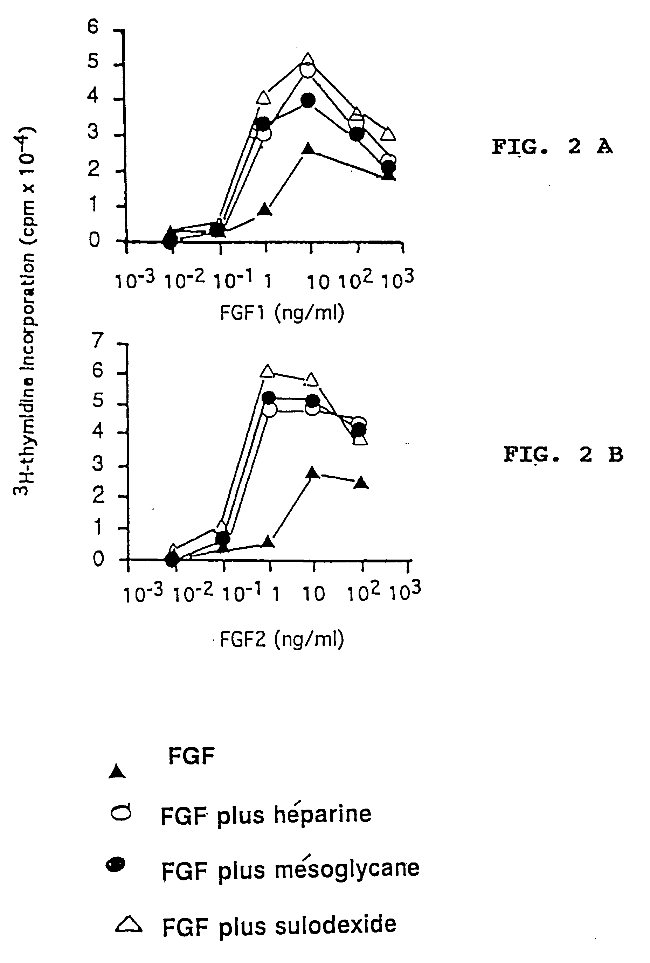

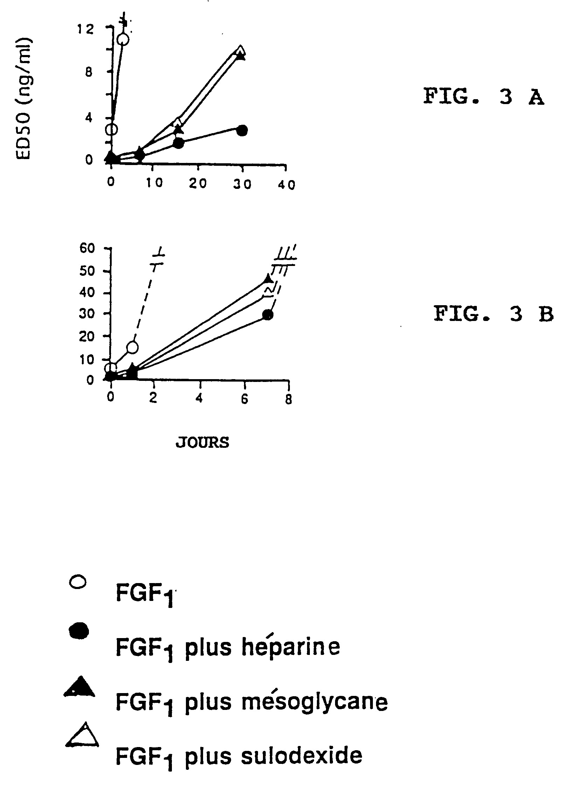

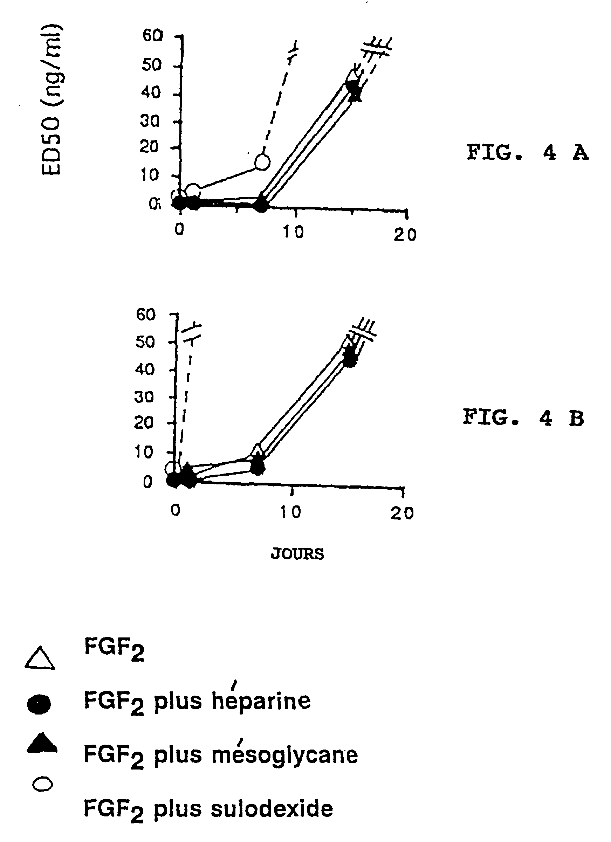

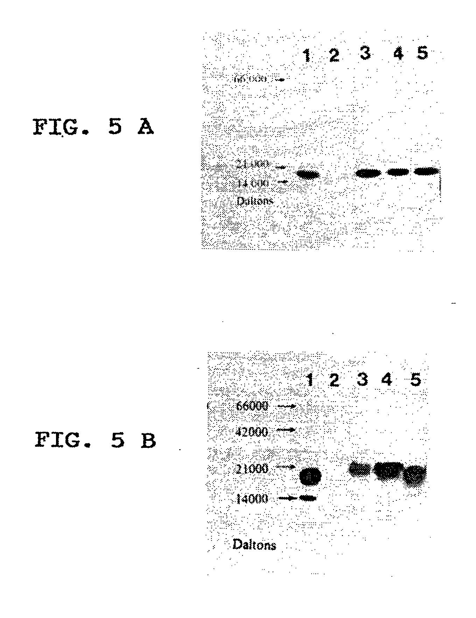

Dans ces expériences le FGF1 ou 2 est utilisé à une dose correspondant à la dose efficace (notée ED50) pour induire une stimulation de l'activité biologique de 50% de la dose induisant la stimulation maximale .L'activité biologique est mesurée par la capacité d'induire une augmentation de l'incorporation de thymidine tritiée dans les cellules selon les protocoles largement décrits dans de nombreuses publications dont celle de Tardieu et coll mentionnée précédemment et également dans le brevet français N°2 644 066. Dans cet exemple l'ED50 est de 5 ng/ml pour le FGF1 et de 3 ng/ml pour le FGF 2, valeurs mesurées expérimentalement (Figs.2a et 2b). La même expérience de stimulation en fonction de la dose de FGF est effectuée en présence de 10 µg/ml de Mésoglycan ou de Sulodexide ou 20 µg/ml d' Héparine. La figure 2 montre que dans ces conditions l'ED50 devient 0.4 ng/ml et 0.2 ng/ml respectivement pour les FGF1 et FGF2 en présence de ces doses de mésoglycan ou d'Héparine. Outre cette capacité à potentialiser l'activité biologique des FGFs les HBGFPP protègent les FGFs contre les dégradations thermiques ainsi que contre l'inactivation induite par l'action protéolytique de la trypsine.(Figs. 3 à 5). De la même manière ces HBGFPP protègent FGF1 et 2 contre une inactivation induite par l'activité protéolytique de la trypsine (Figs. 5a et 5b).

### b) Effets protecteurs des mésoglycans, sulodexides, du dextrane, du dextrane sulfate et de la sucrase vis-à-vis des TGF bêta.

Plusieurs autres composés ont été évalués: le dextrane sulfate (Sigma Chemical, de poids moléculaire 40.000, le dextrane ayant servi à la synthèse du CMDBS (également de chez Sigma) de la sucrase ou sucrose octasulfate (fournie par D. Bar Shalom, Société Bukh Medic, Danemark. Certains de ces composés ont été choisis car ils protègent et stabilisent les FGF. Ainsi, la sucrase (se conférer au brevet US N° 520 2311) ou le dextrane sulfate (se conférer au brevet japonais n° 1 38907/88). Le dextrane est celui qui a servi a la synthèse du CMDBS AM26.

L'expérience de protection de l'activité biologique des TGFbêta a été réalisée de la même manière qu'avec les CMDBS ainsi que décrit dans l'exemple 1 ii: Le mélange d'incubation contient 50 ng de TGF bêta ( dans 0,1 % d'albumine sérique de bovin) et de la trypsine (500 µg). Le mésoglycan ou le sulodexide ou le dextrane sulfate ou le dextrane ou la sucrase sont utilisés à la dose de 5000 µg.

L'activité biologique du TGFbêta est mesurée comme décrit ci-dessus après une dilution de 50 fois et en utilisant des cellules CCL64.

Les résultats sont présentés dans le tableau 4.

Ces résultats illustrent qu'à l'exception de certains CMDBS capables de répondre aux deux critères de sélection vis-à-vis des FGF et TGFbêta seul, parmi les autres composés testés, le mésoglycan présente une activité protectrice significative pour les TGFbêta.

### c) Isolement de la fraction Héparane Sulfate du Sulodexide et du Mésoglycan.

Le Sulodexide et le Mésoglycan correspondent à des mélanges de plusieurs substances dont l'essentiel est constitué de différents glycosaminoglycanes (GAG).

Par une première étape de purification, il a été établi qu'un gramme de produit sec de chacun de ces deux produits contenait respectivement 874 mg pour le mésoglycan et 795 mg pour le sulodexide de GAG totaux.

Cette purification a été obtenue en soumettant ces produits solubilisés à une chromatographie échangeuse d'ions (DEAE - Trisacryl) pour enlever tous les contaminants protéiques. Les GAG totaux ont alors été purifiés en éluant le gel de DEAE avec une solution d'acétate de sodium, pH 4, contenant 1,5 M NaCl.

Après une phase de dialyse extensive contre de l'eau, 60 mg de chaque produit de GAG ont été digérés par la chondroïtinase ABC pendant une nuit à 37°C ( 1 unité par mg de GAG). Cette enzyme dégrade tous les GAG à l'exclusion des héparanes sulfates (HS). Les produits de digestion ont été soumis à une chromatographie sur tamis moléculaire (G50 Sephadex, colonne de 1,8 x 95 cm). L'élution est ensuite réalisée en tampon bicarbonate d'ammonium sous un débit de 18 ml/h. Le matériel non digéré qui correspond à des GAG de nature HS est collecté dans le volume mort d'élution de la colonne.

Les concentrations en GAG sont calculées à partir de leur contenu en acide uronique par la méthode au carbazole (Bitter T. et Muir H.M., 1962, Anal. Biochem 4, 330-334).

Ces dosages ont permis de préciser la composition suivante de chacun des produits:

| | Sulodexide | Mésoglycan |
|---|---|---|
| GAG totaux | 79 % | 87 % |
| Fraction Héparane Sulfate (HS) | 48 % | 52 % |
| Autres GAG | 31 % | 35 % |

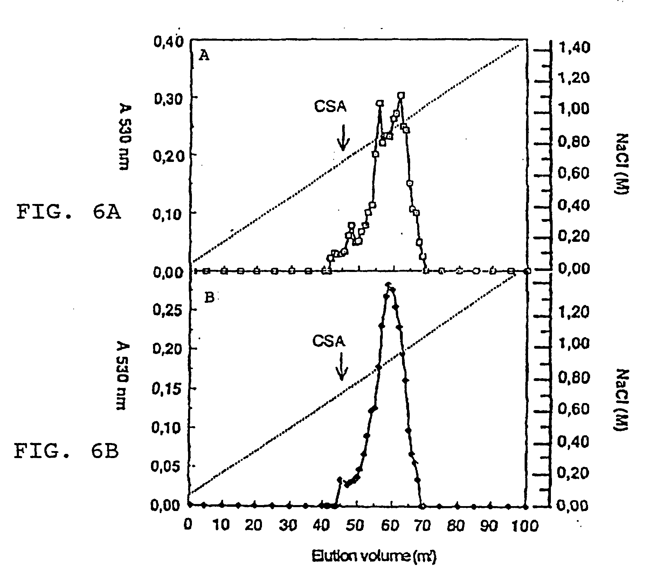

Les fractions HS de chacun de ces deux produits ont été chromatographiées à nouveau sur un gel de DEAE Trisacryl. 1 mg de chaque fraction HS, purifiée à partir du mésoglycan (Fig. 6A) ou du sulodexide ( Fig. 6B), dans 3 ml a été déposé sur une colonne équilibrée avec du tampon 0,05 M NaCI, 0,05 M TMS-Hel pH 7,5. Après un lavage de la colonne par 10 volumes du même tampon suivi d'un lavage par 10 volumes d'un tampon 0,05 M NaCl, 0,05 M d'acétate de sodium pH 4, le matériel fixé à la colonne est désorbé par un gradient salin allant de 0,05 M NaCl à 1,5 M NaCl dans le même tampon acétate. 1 ml de chaque fraction collectée a été dosé par la méthode au carbazole.

Le matériel correspondant aux constituants HS de chacun des produits d'origine présente approximativement le même profil d'élution et donc à peu près la même charge apparente. Ce maximum du pic d'élution est obtenu pour une concentration saline de 0,94 M NaCl. Une fraction définie de chondroïtine sulfate (CSA) a été soumise au même protocole en vue de calibrer la chromatographie. Cette fraction CSA qui ne contient qu'un groupe sulfate par disaccharide est élué à la force ionique de 0,72 M NaCl.

Ces résultats montrent que la fraction HS contient plus de groupements sulfates que les CSA de référence. La fraction HS présente environ deux groupes sulfates par unité dissacharidique.

Ces fractions ont été testées pour connaître leur pouvoir protecteur vis-à-vis du TGF β et du FGF en comparaison des pouvoirs établis avec les produits bruts respectifs.

### Evaluation semi-quantitative des effets protecteurs du FGF par différents polymères.

Comme décrit ci-dessus, une quantité constante de FGF radioactif est incubée dans des conditions différentes. Après autoradiographie des produits de la réaction, la quantité de FGF radioactif non dégradé est quantifiée par densitométrie. Les valeurs correspondent au pourcentage de FGF radiomarqué retrouvé par rapport à la quantité déposée en début de réaction. (Tableau 5).

Les résultats des tableaux 4 et 5 montrent que les fractions HSM et HSS issues respectivement du mésoglycan et du sulodexide présentent des effets protecteurs supérieurs à ces deux compositions et proches de 100%.

### EXEMPLE 3: Effets inhibiteurs in vitro des CMDBS et des glycosaminoplycanes sur l'activité de l'élastase leucocytaire et sur la plasmine.

Les pouvoirs d'inhibition de différents CMDBS et de leurs composés intermédiaires de leur synthèse, ont été établis pour l'élastase leucocytaire et la plasmine.

L'élastase leucocytaire purifiée a été obtenue par Elastin Products Co (Owenville, MO, USA) et la plasmine chez SIGMA.

L'inhibition des activités enzymatiques par ces différents composés est effectuée à 37°C dans un bain thermostaté. Les enzymes considérées sont mises en solution dans un tampon Tris-HCL 100 mM, pH8 pour l'élastase et pH 7,4 pour la plasmine, en présence de 0,02% d'azide de sodium et de 0,01% Triton X100 pour la plasmine. Les concentrations des substrats et celles des enzymes.sont : 0,10 mM MeO-Suc-Ala-Ala-Pro-Val-pNA (paranitroanilide) pour l'élastase à 8,3 nM et 0,20 mM dVal-Leu-dLys-pNA pour la plasmine à 77 nM. Pour chacune des conditions est établi l'IC50.

Le tableau 6 donne les résultats obtenus dans lesquels, le lot AM6 correspond à un dextrane T40 de 40 000 kD. Le lot EM5 correspond à un dextrane T10 de 10 000 kD. Les produits intermédiaires de synthèse sont répertoriés d'après les sigles désignés ci-dessus indexés d'un numéro qui précise le nombre de chacune des réactions de substitution.

Les valeurs des IC50 démontrent que les CMDBS ont des effets inhibiteurs de type hyperbolique non compétitif sur l'activité de l'élastase leucocytaire comparables à ceux de l'héparine, l'un des meilleurs inhibiteurs de cette activité (Ki de l'ordre de 1 nM). Les CMDBS exercent de plus et à l'inverse de l'héparine des effets inhibiteurs sur la plasmine.

Il ressort en outre du tableau 6 que les effets inhibiteurs des fractions HSM et HSS sont supérieurs à ceux du mésoglycan et du sulodexide, respectivement.

### Exemple 4 : Effet du CMDBS sur la réinnervation du muscle squelettique du rat adulte au cours de la régénération post-traumatique.

### INTRODUCTION:

Des travaux antérieurs ( Schultz E. Anat. Rec.1984, 208, 501-506 ; Bassaglia Y. Thèse de l'Université Paris-XII, Créteil, 1991 ) ont montré que les muscles lents posturaux en régénération ( type Soleus ) présentaient un défaut de réinnervation motrice en relation avec des altérations des lames basales musculaires. Ces lames basales jouent le rôle d'un interface fonctionnel au niveau de la synapse. Les axones moteurs de ces muscles ne parviennent pas à retrouver leurs anciennes cibles et les fibres musculaires non réinnervées subissent une dégénéscence fibrotique deux mois après la lésion.

Ce phénomène involutif post-traumatique ne se produit pas dans les muscles phasiques rapides du type de l'Extensor digitorum longus ( EDL ) dont les fibres récupèrent leur fonctionnalité au bout de deux mois.

Un analogue de synthèse des héparanes matriciels, le CMDBS ( carboxyméthyldextrane benzylamide sulfonate ) s'est révélé particulièrement efficace pour accroître la vitesse et le degré de régénération des deux types de muscles et tout particulièrement le soléaire. Le CMDBS a été expérimenté sur la réformation des plaques motrices de muscles en régénération, traités ou non par le CMDBS, dans un intervalle de temps de 1 à 60 jours.

L'étude histologique de la réinnervation a été effectuée en révélant cytochimiquement l'acétylcholinestérase ( AChE ), ( Gautron J. Histochem., 1982, 76,469-478 ) des plaques motrices combinée a une méthode d'imprégation argentique des axones ( Hopkins W.G., J. Physiol. 1981, 320, 5-6 ) dans des coupes de muscles.

Parmi les principales isoformes de l'AChE ( 4 S, 10S et 16S ), la forme asymétrique dodécamérique 16S est concentrée à la synapse neuro-musculaire, chez le rat. Elle se lie par interaction ionique à la basale par l'intermédiaire d'une triple hélice de collagène ( Vigny M., Koenig J. and Rieger F. J. Neurochem., 1976, 27, 1347-1353; Blondet B et Gautron J. Biol. Cell., 1980, 38, 203-210; Blondet B., Rieger F., Gautron J. and Pinton-Raymond M. Dev. Biol. 1986, 117, 13-23 ). Cette isoforme constitue un témoin biochimique sensible de modifications moléculaires très fines des lames basales synaptiques.

Le degré de réinnervation des muscles régénérés a été mesuré en dosant la choline acétyl transférase. Cette enzyme de synthèse du médiateur chimique dans les boutons terminaux constitue un marqueur spécifique de la croissance axonale à l'intérieur du muscle. L'élévation de son activité accompagne notamment le bourgeonnement des axones.

### MATERIEL ET METHODES

### 1- Lésion des muscles squelettiques:

45 rats wistar mâles de deux mois et demi répartis en deux lots traités parallèlement pour les études histologiques sur coupes ou biochimiques après homogénéisation ont été utilisés.

Les rats ont été anesthésiés par l'éther. Les muscles EDL et Soleus ont été lésés par écrasement à l'aide d'une pince de Péan dont la pression a été maintenue 10 secondes environ pour léser les fibres au milieu du muscle. Les nerfs moteurs ont été préalablement sectionnés, à l'entrée du muscle, afin d'éviter une lésion irreproductible des axones lors de l'écrasement du muscle. 100 µl de CMDBS à 50 µg / ml, réchauffé à 37°C, ont été injectés en 20 secondes dans le muscle à l'aide d'une microseringue Hamilton munie d'une aiguille de 50mm de long et 0,3 mm de diamètre Les muscles controlatéraux non lésés ont été utilisés comme témoins afin d'annuler les variation individuelles.

Le réveil des rats après anesthésie se fait en 10 mn. Ils récupèrent une activité motrice en 30 mn. Les rats opérés ont été mis dans des cages individuelles de 6 dm2 et nourris à volonté.

### 2- Cytochimie des AChE

L'enzyme a été révélée par la méthode de J. Gautron (Hopkins W.G., J. Physiol. , 1981, 320, 5-6). Les muscles fixés par le formaldéhyde à 4% ont été coupés longitudinalement à 80 mm d'épaisseur à l'aide d'un microtome à congélation. Après rinçage, les coupes ont été incubées pendant 30mn dans le milieu de révélation contenant 50ml d'acétyl-sulfure (Aldrich ref.:A2,220-3 ), 1ml de Pb(NO3) à 3% dans l'eau, 50 mg d'acétylthiocholine bromure (Aldrich ref.85,533-2 ) dans 100ml de tampon tris-maléate-NaOH 0,2M, pH 6,8.

### 3-Imprégnation argentique des axones

Après révélation de l'AChE, les coupes longitudinales sont déshydratées par l'éthanol 100% puis réhydratées dans l'eau distillée et incubées pendant 30mn dans la solution argentique contenant 0,31g d'acide borique, 19mg de borax, 2,5g d'AgNO3 pour 250ml d'eau. La coloration est développée pendant 10mn dans une solution de 5g de sulfite de sodium, 1g d'hydroquinone, 0,76g de borax pour 100ml d'eau. Après rinçage à l'eau, les coupes sont déshydratées par l'éthanol, éclaircies par le xylène et montées entre lame et lamelle dans le Baume de Canada.

Le nombre de plaques motrices a été estimé par comptage dans chaque coupe longitudinale de muscle.

### 4- Séparation des isoformes de l'acétylcholinestérase

Une deuxième série de muscles témoins et en régénération a été broyée à 300 RPM dans un homogénéiseur de Potter verre/verre contenant 0,5ml de solution d'extraction: 1% de Triton X100, 1M Nacl, 1mM EGTA et 10mM tampon tris/Hcl pH 7. Les homogénats sont centrifugés pendant 20mn à 20.000g et 100ml du surnageant est déposé sur chaque gradient de densité.

La séparation des isoformes est effectuée sur gradient continu et linéaire de sucrose 5 à 20% contenant les mêmes concentrations de Triton, NaCl, EGTA et tampon que l'homogénat. Après 17h de centrifugation à 38.000 RPM dans un rotor Kontron TST 41. 14, chaque gradient a été réparti en 40 fractions de 0,3ml environ. Chaque fraction a été additionnée de 1,5ml de réactif d'Ellman; l'activité AChE a été mesurée à 412nm, après 4h d'incubation.

La proportion de la forme asymétrique 16S a été mesurée en % de l'activité totale de chaque gradient.

### 5- Dosage de la Choline acétyl transférase ( CAT ).

Les muscles ont été homogénéisés dans du tampon tris/HCl 0,1M pH 7,6, contenant 2mM d'EDTA. Les homogénats ont été centrifugés à 13.000g pendant 6 mn. L'activité de la CAT a été mesurée dans les surnageants par la méthode de Rand et Jonhson ( Rand J.B. and Johnson C.D. Anal. Biochem., 1981, 116,361-371 ).

L'incubation se fait en présence de choline tritiée (1,5 mCi ), de 5 mM d'acétyl CoA et de 200 mM de bromure de néostigmine dans un tampon tricine/NaOH 0,1M pH 8. La choline non acétylée est ensuite phosphorylée par la choline kinase (5 µ ) en présence de 0,1M. d'ATP, 0,2M. MgCl2 pour 1,25 ml de tampon tris 0,1M. pH 8,1. L'acétyl-3H choline synthétisée est mesurée dans un liquide scintillant pour phase organique contenant 3mg/ml de tétraphényl borate

### RESULTATS

### 1- Etude histologique et cytochimique

Les fibres rapides ( EDL ) possèdent une gouttière synaptique plus développée que celle des fibres lentes ( figs. 7A et 7D ); les plis sous-neuraux sont également plus développés.

Les muscles adultes lésés et non traités par le CMDBS régénèrent complètement en 60 jours environs (fig. 7B ). Les plaques motrices, avec différenciation de la gouttière synaptique, sont bien visibles et sont innervées par les axones. On remarque cependant, qu'après 60 jours de régénération, la surface de la gouttière est plus réduite que celle des témoins controlatéraux non lésés (Figs. 7A et 7B ).

L'injection d'une dose unique de CMDBS ( 100 µl à 50µg/ml ), au moment de la lésion, augmente nettement la surface synaptique. Après 17 jours, les muscles EDL traités montrent une gouttière très différenciée et innervée dont la surface est supérieure ( environ 150% ) à celle des témoins non lésés ( figs. 7A et 7C ).

Les fibres lentes ( 85% dans le soleus ) ont des plaques motrices de diamètre comparable à celles de l'EDL mais avec une surface synaptique plus réduite ( fig.7D ) et les terminaisons nerveuses y sont moins nombreuses.

En l'absence de traitement par le CMDBS, le Soleus régénére très incomplètement. Après 60 jours (fig. 7E ), les plaques ont un aspect diffus, non différencié, probablement dû à une réinnervation incomplète ou absente, l'imprégnation argentique ne montre pas clairement la présence d'axones. On observe par contre de nombreux bourgeonnements anarchiques des axones à l'intérieur du muscle.

Le traitement par le CMDBS améliore considérablement la reformation des plaques motrices (fig. 7F ) qui présentent des gouttières différenciées et comparables à celles des témoins. Les axones moteurs sont également visibles à proximité et au niveau des plaques.

Ces observations ont été quantifiées en comptant les plaques par coupe longitudinale de muscle ( fig. 8 ).Les deux muscles montrent une évolution très différente: dans l'EDL, 80% des plaques disparaissent au bout de 3 jours de régénération, puis leur nombre augmente régulièrement de 7 à 45 jours pour atteindre 90% du témoin. Dans le Soleus,la diminution initiale est moins importante, l'augmentation est ensuite plus rapide mais, au contraire de l'EDL, ce nombre diminue après 13 jours et se stabilise à 60% du témoin. Cette augmentation transitoire des plaques entre 7 et 13 jours peut être dûe à la formation de synapses temporaires ( synapses ectopiques ), les axones ne réoccupant pas leur ancienne gouttière au niveau des basales synaptiques.

### 2- Etude biochimique

### 2-1- Muscles régénérés et non traités.

Les observation précédentes, ainsi que celles issues de l'immunocytochimie de la laminine et de l'activité protéolytique , laissent penser que, dans le cas du soleus, les structures matricielles pourraient être partiellement altérées, notamment celles des basales synaptiques par suite d'altérations protéasiques lors de la myolyse initiale. L'acétylcholinestérase 16S (dodécamère asymétrique A12 ) est focalisée chez le rat au niveau des lames basales synaptiques ( Vigny et al. précédemment cités) et constitue un marqueur très sensible de l'évolution de ces structures.

Au cours de la régénération, les deux muscles présentent des évolutions différentes de la proportion de cette isoforme (fig. 9 ). Dans l'EDL, après une chute initiale de 85% elle augmente régulièrement pour atteindre 90% du témoin non lésé après 28 jours de régénération.

Dans le soleus, la chute initiale est plus importante (98% ), elle remonte rapidement jusqu'au 13eme jour mais elle décroît ensuite fortement pour atteindre 20% seulement après 28 jours.

Ces deux résultats confirment les observations histologiques de l'évolution du nombre de plaques (fig. 8 ).

### 2-2- Muscles régénérés après traitement par le CMDBS.

La croissance axonale à l'intérieur du muscle et la réinnervation ont été suivies par la mesure de l'activité de la choline acétyl-transférase ( CAT) (figs. 10 et 11 ).

En comparant l'activité CAT dans les deux muscles après 7 jours de régénération, on observe que le CMDBS diminue celle-ci par rapport au muscle homologue non traité. Cet effet est particulièrement marqué dans le cas du soleus. A ce stade on observe fréquemment un bourgeonnement axonal dans les coupes histologiques. A 13 jours les valeurs de Cat dans les EDL traités ou non sont comparables mais à 16 jours, l'activité Cat est 2 fois plus élevée dans l'EDL traité. Ceci peut être rapproché des observations histologiques qui montraient une augmentation de la surface synaptique après traitement.

Dans le cas du soleus, l'évolution après 13 jours est différente: le muscle traité ne subit pas la chute secondaire d'activité qui se produit en l'absence de CMDBS ( fig. 11 ).

En conclusion, l'ensemble cohérent des résultats histologiques et biochimiques met en évidence que le CMDBS agit très efficacement sur la régénération axonale et synaptique du muscle strié. Cet effet, particulièrement important dans le muscle lent postural, est obtenu après une injection unique aussitôt après la lésion. Cette unique traitement suffit pour influencer toute la régénération ultérieure.

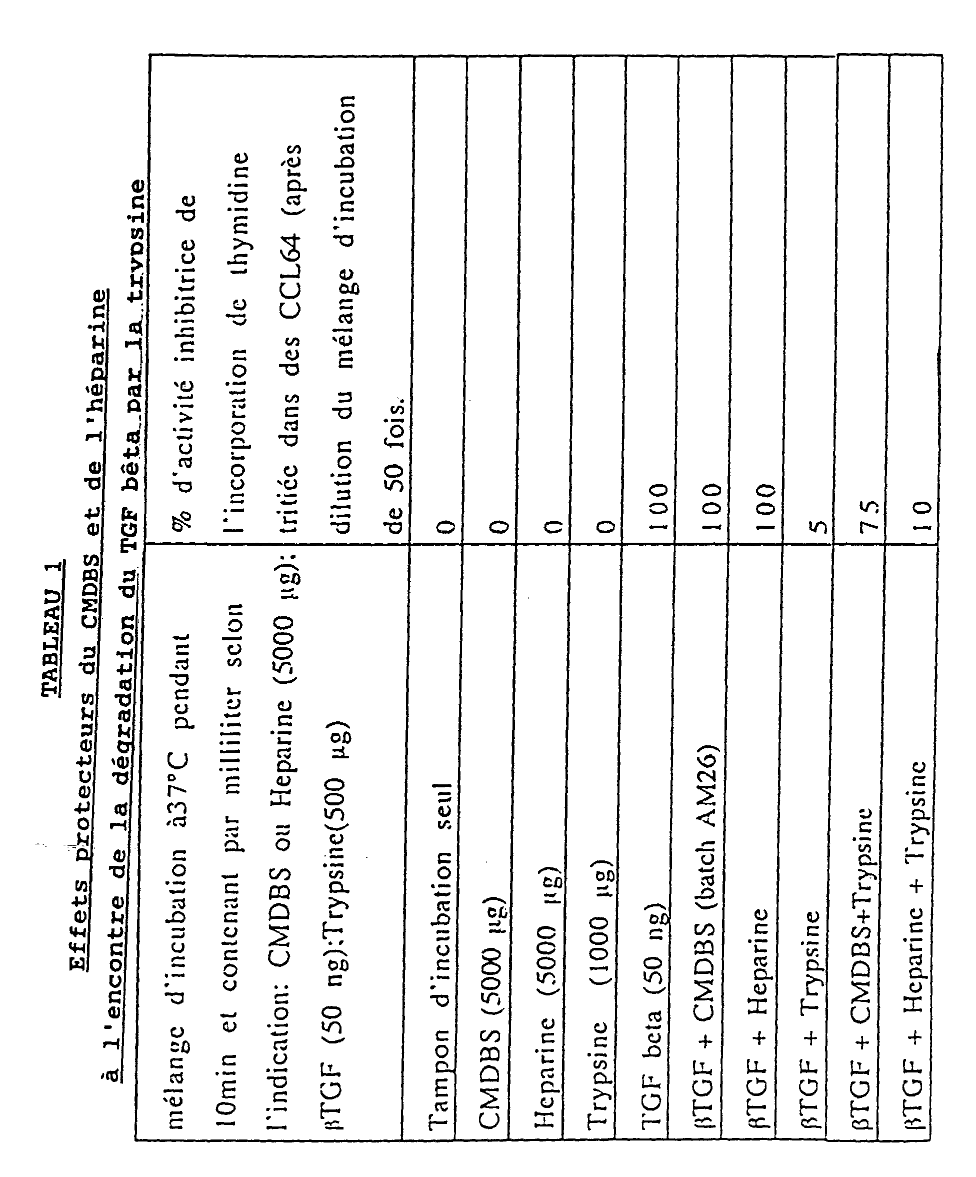

**TABLEAU 2**

| **Effet non inhibiteur du CMDBS vis-à-vis de la trypsine** | |
|---|---|
| Trypsine (10ug/ml)+ S87 | 1.00 |
| Trypsine+S87+5ug/ml CMDBS | 100 |
| Trypsine+S87+50ug/ml CMDBS | 100 |
| Trypsine+S87+500ug/ml CMDBS | 100 |
| Trypsine+S87+STB1 | 0 |

**TABLEAU 3 :**

| Origine , activité anticoagulante et composition partielle du Mésoglycan et du Sulodexide (informations du fournisseur | | |
|---|---|---|
| | Sulodexide | Mésoglycan |
| Origine | duodenum de porc | aorte |
| Activité anticoagulante | 50-70 IU/mg | < 50 IU /mg |
| Composition chimique | | |
| Dermatane sulfate | 20 - 35 % | 25 - 60 % |
| Chondroitine Sulfate | 2-7% | 3-15% |
| Héparane sulfate | + | + |

**TABLEAU 4**

| Protection du TGFbêta par divers polymères | |
|---|---|
| TGF bêta | 100 % |
| TGF bêta + trypsine | 0 % |
| TGF bêta + mésoglycan | 100 % |
| TGF bêta + mésoglycan + trypsine | 50 % |
| TGF β + HSM | 100 % |
| TGF β + HSM + trypsine | 75 % |
| TGF beta + sulodexide | 100 % |
| TGF beta + sulodexide + trypsine | 20 % |
| TGF β + HSS | 100 % |
| TGF β + HSS + trypsine | 45 % |
| TGF bêta + Dextrane | 100 % |
| TGF bêta + Dextrane + trypsine | 0 % |
| TGF bêta + Dextrane Sulfate | 100 % |
| TGF bêta + Dextrane Sulfate + trypsine | 0 % |
| TGF bêta + Sucrase | 100 % |
| TGF bêta + Sucrase + trypsine | 0 % |
| HSM = Héparanes Sulfates purifiés à partir du Mésoglycan | |
| HSS = Héparanes Sulfates purifiés à partir de Sulodexide | |

**TABLEAU 5**

| Protection du FGF par divers polymères | |
|---|---|
| | PROTECTION EN % |
| FGF seul | 100 |
| FGF + Trypsine | 0 |
| FGF + Trypsine + Héparine | 100 |
| FGF + Trypsine + Mesoglycan | 75 |
| FGF + Trypsine + Sulodexide | 70 |
| FGF + Trypsine + Mesoglycan traité Heparinase | 0 |
| FGF + Trypsine + Sulodexide traité Héparinase | 0 |
| FGF + Trypsine + Héparine Traité Héparinase | 0 |
| FGF + HSM + Trypsine | 95 |
| FGF + HSS + Trypsine | 90 |

**TABLEAU 6**

| Inhibition des activités de l'élastase et de la plasmine | | |
|---|---|---|
| Composés testés | Elastase Leucocytaire | Plasmine |
| | IC₅₀ en µg/ml | IC₅₀ en µg/ml |
| CMDBS lot AM6 | 2,2 | 1,5 |
| T40 | > 100 | > 100 |
| CMDBS lot EM5 | 10 | 7 |
| T10 CMD2B | 50 | 53 |
| T10 5CMD1B | > 100 | > 100 |
| T10 3CMD | > 100 | > 100 |
| T10 | > 100 | > 100 |
| Mesoglycan | 72 | 65 |
| HS Mesoglycan | 20 | 22 |
| Sulodexide | 79 | 75 |
| HS Sulodexide | 25 | 20 |
| Héparine | 1,8 | |
| Lipo-héparine | | 0,5 |
| HSM = Héparanes Sulfates purifiés à partir du Mesoglycan | | |
| HSS = Héparanes Sulfates purifiés à partir de Sulodexide | | |

## Revendications

1. Utilisation d'au moins un polymère ou un biopolymère, appelés HBGFPP, protégeant spécifiquement les facteurs de croissance des familles des FGF et TGF bêta de la dégradation trypsique et n'inhibant pas de manière significative la coagulation, pour la fabrication d'un médicament pour le traitement des lésions du système nerveux.

2. Utilisation selon la revendication 1, **caractérisée en ce que** le polymère ou biopolymère présente une activité anti-coagulante inférieure à 50 u.i. par mg de polymère.

3. Utilisation selon l'une des revendications 1 et 2 , **caractérisée en ce que** ledit polymère n'active substantiellement pas le système du complément.

4. Utilisation selon l'une des revendications 1 à 3, **caractérisée en ce que** ledit polymère potentialise in vitro les FGF.

5. Utilisation selon l'une des revendications 1 à 4, **caractérisée en ce que** ledit polymère inhibe substantiellement lès activités protéasiques de l'élastase et/ou de la plasmine.

6. Utilisation selon l'une des revendications 1 à 5, **caractérisée en ce que** ledit polymère ou biopolymère est un polysaccharide.

7. Utilisation selon la revendication 6 , **caractérisée en ce que** ledit polysaccharide est principalement composé de résidus glucose.

8. Utilisation selon la revendication 6, **caractérisée en ce que** le polysaccharide comprend des résidus glucosamine et/ou d'acide uronique.

9. Utilisation selon la revendication 8, **caractérisée en ce que** le polysaccharide comprend des dimères glucosamine-acide uronique.

10. Utilisation selon l'une des revendications 8 et 9, **caractérisée en ce que** ledit polysaccharide est un glycosaminoglycane éventuellement associé à un lipide, un peptide ou un protide, ou un sulfate d'un de ces composés.

11. Utilisation selon l'une des revendications 1 à 6, **caractérisée en ce que** ledit polysaccharide est un dextrane substitué.

12. Utilisation selon la revendication 11, **caractérisée en ce que** ledit polysaccharide est un dextrane substitué par des résidus carboxyméthyle, benzylamine et sulfonate (CMDBS).

13. Utilisation selon l'une des revendications 1 à 5, **caractérisée en ce que** ledit polymère est de nature non-osidique.

14. Composition pharmaceutique pour le traitement des lésions du système nerveux contenant au moins un polymère tel que défini dans l'une des révendications 1 à 13 à l'exception du mesoglycan et du sulodexide en association avec au moins un excipient pharmacologiquement acceptable.

## Patentansprüche

1. Verwendung mindestens eines Polymers oder eines Biopolymers mit der Bezeichnung HBGFPP, das insbesondere die Wachstumsfaktoren der FGFund der β-TGF-Familie vor dem Trypsinabbau schützt und die Gerinnung nicht signifikant hemmt, zur Herstellung eines Medikaments zur Behandlung von Läsionen des Nervensystems.

2. Verwendung nach Anspruch 1, **dadurch gekennzeichnet, dass** das Polymer oder Biopolymer eine gerinnungshemmende Aktivität von weniger als 50 I.E. pro mg Polymer aufweist.

3. Verwendung nach einem der Ansprüche 1 und 2, **dadurch gekennzeichnet, dass** das Polymer das restliche System im wesentlichen nicht aktiviert.

4. Verwendung nach einem der Ansprüche 1 bis 3, **dadurch gekennzeichnet, dass** das Polymer die FGF in vitro potenziert.

5. Verwendung nach einem der Ansprüche 1 bis 4, **dadurch gekennzeichnet, dass** das Polymer die Proteasenwirkungen von Elastase und/oder von Plasmin im wesentlichen hemmt.

6. Verwendung nach einem der Ansprüche 1 bis 5, **dadurch gekennzeichnet, dass** das Polymer oder Biopolymer ein Polysaccharid ist.

7. Verwendung nach Anspruch 6, **dadurch gekennzeichnet, dass** das Polysaccharid hauptsächlich aus Glucoseresten besteht.

8. Verwendung nach Anspruch 6, **dadurch gekennzeichnet, dass** das Polysaccharid Glucosamin- und/oder Uronsäurereste umfasst.

9. Verwendung nach Anspruch 8, **dadurch gekennzeichnet, dass** das Polysaccharid Glucosamin-Uronsäure-Dimere umfasst.

10. Verwendung nach einem der Ansprüche 8 und 9, **dadurch gekennzeichnet, dass** es sich bei dem Polysaccharid um ein Glykosaminoglycan handelt, das gegebenenfalls mit einem Lipid, einem Peptid oder einem Proteid oder einem Sulfat dieser Verbindungen verbunden ist.

11. Verwendung nach einem der Ansprüche 1 bis 6, **dadurch gekennzeichnet, dass** es sich bei dem Polysaccharid um ein substituiertes Dextran handelt.

12. Verwendung nach Anspruch 11, **dadurch gekennzeichnet, dass** es sich bei dem Polysaccharid um ein Dextran handelt, das mit Carboxymethyl-, Benzylamin- und Sulfonatresten (CMDBS) substituiert ist.

13. Verwendung nach einem der Ansprüche 1 bis 5, **dadurch gekennzeichnet, dass** das Polymer eine Nicht-Glycosid-Beschaffenheit hat.

14. Pharmazeutische Zusammensetzung zur Behandlung von Läsionen des Nervensystems, enthaltend mindestens ein Polymer gemäß der Definition in einem der Ansprüche 1 bis 13 mit Ausnahme von Mesoglycan und Sulodexid in Verbindung mit mindestens einem pharmazeutisch annehmbaren Trägermittel.

## Claims

1. Use of at least one polymer or one biopolymer, called HBGFPP, specifically protecting the growth factors of FGFs and beta TGFs families from tryptic degradation and not significantly inhibiting coagulation, in the manufacture of a drug for the treatment of lesions of the nervous system.

2. Use according to claim 1, **characterized in that** the polymer or biopolymer presents an anticoagulant activity of less than 50 international units per mg of polymer.

3. Use according to one of claims 1 and 2, **characterized in that** the said polymer does not substantially activate the complement activity.

4. Use according to one of claims 1 to 3, **characterized in that** the said polymer potentializes the FGFs in vitro.

5. Use according to one of claims 1 to 4, **characterized in that** the said polymer substantially inhibits the proteasic activities of elastase and/or plasmin.

6. Use according to one of claims 1 to 5, **characterized in that** the said polymer or biopolymer is a polysaccharide.

7. Use according to claim 6, **characterized in that** said polysaccharide is primarily composed of glucose residues.

8. Use according to claim 6, **characterized in that** the polysaccharide comprises glucosamine and/or uronic acid residues.

9. Use according to claim 8, **characterized in that** the polysaccharide comprises glucosamine-uronic acid dimers.

10. Use according to one of claims 8 and 9, **characterized in that** the said polysaccharide is a glycosaminoglycan, possibly associated with a lipid, a peptide or a protide, or a sulfate of one of these compounds.

11. Use according to one of claims 1 to 6, **characterized in that** the said polysaccharide is a substituted dextran.

12. Use according to claim 11, **characterized in that** the said polysaccharide is a dextran substituted by carboxymethyl, benzylamine and sulfonate residues (CMDBS).

13. Use according to one of claims 1 to 5, **characterized in that** the said polymer is of non-osidic nature.

14. Pharmaceutical composition for the treatment of lesions of the nervous system containing at least one polymer such as defined in claims 1 to 13, excluding mesoglycan and sulodexide in association with at least one pharmacologically acceptable excipient.
